# EUROPÄISCHE PATENTSCHRIFT

(11) **EP 3 365 043 B1**
(45) Veröffentlichungstag und Bekanntmachung des Hinweises auf die Patenterteilung: **23.09.2020**
(21) Anmeldenummer: 16785131.0
(22) Anmeldetag: 20.10.2016
(51) Int. Cl.: A61M 1/16, A61M 1/36, A61B 5/145, A61B 5/1455, G01N 33/49

(54) **ZWISCHENELEMENT FÜR EINE MEDIZINTECHNISCHE EXTRAKORPORALE FLUIDLEITUNG**
INTERMEDIATE ELEMENT FOR A MEDICAL EXTRACORPOREAL FLUID CONDUIT
ÉLEMENT INTERMEDIAIRE POUR UNE CONDUITE DE FLUIDE EXTRACORPOREL MEDICAL

(30) Priorität: 23.10.2015 EP 15003041
(43) Veröffentlichungstag der Anmeldung: 29.08.2018
(73) Patentinhaber: Novalung GmbH, 74076 Heilbronn (DE)
(72) Erfinder: MAURER, Andreas, 72072 Tübingen (DE); FILIPON, Sven, 74080 Heilbronn (DE)
(74) Vertreter: Graf von Stosch Patentanwaltsgesellschaft mbH
(86) Internationale Anmeldenummer: PCT/EP2016/075294
(87) Internationale Veröffentlichungsnummer: WO 2017/068081

(56) Entgegenhaltungen:
- EP-A1- 0 993 266
- US-A- 4 425 918
- US-A- 4 734 184
- US-A- 5 387 329
- US-A1- 2012 258 545

## Beschreibung

Die vorliegende Erfindung betrifft ein Zwischenelement für eine medizintechnische extrakorporale Fluidleitung, die dazu eingerichtet ist, Blut zu führen, mit einem Basiskörper, der sich zwischen Verbindungsteilen erstreckt, wobei Basiskörper und Verbindungsteile durchgehend von einem Durchflusskanal zur Führung des Fluid durchsetzt sind und wobei die Verbindungsteile dazu eingerichtet sind, den Basiskörper zum Leiten des Blutes mit der Fluidleitung zu verbinden und wobei peripher am Basiskörper eine Aufnahme angeordnet ist, die dazu eingerichtet ist, einen Messwertgeber aufzunehmen und wobei an der Aufnahme im Basiskörper ein Durchbruch zum Durchflusskanal angeordnet ist, der über ein elastisches Element zur Aufnahme hin fluiddicht verschlossen ist, wobei die Aufnahme dazu eingerichtet ist, einen als Gassensor ausgebildeten Messwertgeber einer Sensoreinrichtung zur Messung wenigstens eines in dem Blut enthaltenen Gases aufzunehmen, und wobei das elastische Element ein Diffusionselement ist, welches permeabel für wenigstens ein Gas ist, wobei das Diffusionselement in einem Verbindungsbereich stoffschlüssig mit einem Rand des Durchbruchs verbunden ist und als Diffusionsfolie von kreisrunder Geometrie ausgebildet und zur Aufnahme hin vorgespannt ist. Ferner betrifft die vorliegende Erfindung ein medizintechnisches extrakorporales Fluidsystem, insbesondere einen medizintechnischen extrakorporalen Blutkreislauf, mit wenigstens einer Fluidleitung, die dazu eingerichtet ist, Blut zu führen, mit wenigstens einem Einleitelement, das dazu eingerichtet ist, Blut von einem Patienten in die Fluidleitung zu führen und mit einem Gasaustauschmodul, das hydraulisch mit der wenigstens einen Fluidleitung verbunden ist, wobei in die Fluidleitung ein erfindungsgemäßes Zwischenelement hydraulisch zwischengeschaltet ist.

Die vorliegende Erfindung betrifft insbesondere ein Zwischenelement mit einzelnen Merkmalen des Anspruchs 1, sowie ein medizintechnisches extrakorporales Fluidsystem mit einzelnen Merkmalen des Anspruchs 12.

Es sind Zwischenelemente für medizintechnische extrakorporale Fluidleitungen, z.B. aus Systemen für medizintechnische extrakorporale Lungenunterstützung (ECLA/ILA), bekannt. Derartige Zwischenelemente verbinden dabei als Konnektoren Fluidleitungen, wie z.B. Schlauchleitungen aus z.B. PVC, oder sie sind als Adapter ausgebildet, die dazu dienen, verschiedenartige Fluidleitungen miteinander zu verbinden oder diese Fluidleitungen an andere Komponenten des Systems anzubinden. Weitere Systeme, in denen beispielhaft derartige Zwischenelemente zum Einsatz kommen, sind Systeme zur extrakorporalen Membranoxigenation (ECMO), Systeme zur pumpenlosen arterio-venösen Lungenunterstützung (PECLA) oder Systeme zur parakorporalen Lungenunterstützung (PLA). Die Anwendungsgebiete derartiger Systeme umfassen z.B. ARDS (akutes Lungenversagen), ALI (akute Lungenverletzung), Nirentherapie, bei Patienten mit chronischen Lungenversagen (COPD/AECOPD) oder auch bei Lungenersatzverfahren während Operationen am Herzen. Ein Einsatz im Labor, für eine schnelle Analyse für die Parameter CO₂, O₂ ist ebenfalls denkbar.

Derartige Zwischenelemente kommen außerdem in Systemen zur extrakorporalen Kreislaufunterstützung bzw. extrakorporalen Lifesupport (ECLS) zur Anwendung. Anwendungsgebiete der ECLS sind beispielsweise Patienten mit Krankheitsbildern in der Kardiologie und Kardiochirurgie, z.B. bei Patienten mit einer Dekompensation einer Herzinsuffizienz, bei denen die Herzfunktion nicht mehr dazu ausreicht, den Organismus ausreichend mit sauerstoffreichem Blut zu versorgen.

Bei derartigen Systemen muss das dem System zugeführte Blut in der Regel aufwendig über einen Zugang an der Fluidleitung entnommen und in einer Blutgasanalyse (BGA) auf den O₂- und CO₂ -Gehalt überprüft werden. Diese Prozedur beansprucht viel Zeit und ist aufwendig. Alternativ besteht derzeit nur die Möglichkeit transkutane CO₂/O₂₋Messungen auf der Haut des Patienten durchzuführen. Dazu ist es notwendig, einen Messsensor an der Haut des Patienten anzulegen und den Spalt zwischen der Haut und dem Sensor mit einer Flüssigkeit auszufüllen, damit die Messung überhaupt durchgeführt werden kann. Dieses Verfahren ist jedoch ebenfalls sehr aufwendig, da eine Kalibrierung und Temperierung (41 - 43 °C) notwendig ist aufgrund der Mikrozirkulation der Haut.

Aus der DE 10 2013 012 433 A1 ist eine Anordnung für eine extrakorporale Kreislaufunterstützung bekannt, bei der ein extrakorporaler Blutkreislauf von einer venösen Kanüle zu einer arteriellen Kanüle über einen Oxygenator erfolgt, indem das Blut mit Sauerstoff angereichert und CO₂ entfernt wird. Eine im extrakorporalen Blutkreislauf angeordnete Blutpumpe fördert dabei Blut über Leitungen von der venösen Kanüle zur arteriellen Kanüle. Eine Messung des O₂- bzw. CO₂-Gehalts des oxygenierten Blutes ist dieser Schrift nicht zu entnehmen.

Aus der WO 2012/127422 A1 ist eine Vorrichtung für die extrakorporale Überwachung der physikalisch-chemischen Parameter von Blut bekannt, die einen länglichen Körper mit einem Einlass und einem Auslass für organische fließende Medien aufweist. In dem Körper ist eine Detektionskammer zwischen dem Einlass und dem Auslass angeordnet, durch welche die organische Flüssigkeit fließt. Der Körper weist dabei zumindest ein erstes Gehäuse für ein erstes Sensorelement zum Abtasten eines Druckparameters des Fluids und wenigstens ein zweites Gehäuse für ein zweites Sensorelement zum Erfassen eines anderen Parameters als dem Druckparameter auf. Beispielhaft können an den Gehäusen Sauerstoffdrucksensoren und CO₂-Drucksensoren angeordnet werden. Dabei weist der Sensor eine hydrophobe und elastisch deformierbare Membran auf, die dazu designet ist, in Kontakt mit der zu überwachenden Flüssigkeit gebracht zu werden.

Aus der WO 97/15228 A1, von der die vorliegenden Erfindung ausgeht, ist ferner ein Kunststoffelement bekannt, welches der Verbindung von zwei Schläuchen dient. In einer Öffnung des Kunststoffelements ist ein flexibles Diaphragma angeordnet, welches die Öffnung in der Seitenwand des Plastikelements fluiddicht verschließt. An die Öffnung schließt sich ein Verbindungsarm an, in welchem ein Verbindungsgehäuse mit einem Messwertgeber einsteckbar ist. In eingestecktem Zustand befindet sich zwischen dem Messwertgeber und dem flexiblen Diaphragma ein mit Luft gefüllter Raum. Die Anordnung dient dabei der Messung des Blutdrucks. Für eine Messung der Gasanteile in einem Fluid ist dieses Element hingegen nicht geeignet.

Die Druckschrift US 2012/258545 A1 beschreibt ein Verfahren zur Feststellung des Bicarbonat-Gehalts in einer Dialyseflüssigkeit, der auf der Grundlage der Kohlendioxidmenge sowie des pH-Werts berechnet wird, und entsprechende Dialysesysteme. In solchen Dialysesystemen kann die Dialyseflüssigkeit eine Dialysatsammeleinheit durchfließen, bevor sie in die Hämodialyse-Maschine eintritt. Die Dialysatsammeleinheit dient der Messung des pH-Werts sowie des Kohlendioxidpartialdrucks der Dialyseflüssigkeit.

Die Druckschrift EP 0 993 266 A1 offenbart eine Kassette zur Messung von einem oder mehreren Parametern im Blut, die mit einer Messeinrichtung verbindbar ist. Diese Kassette umfasst wenigstens einen Sensor zur Messung eines Blutparameters sowie eine speziell ausgeführte Aufnahme für die Messeinrichtung.

Die Druckschrift US 5 387 329 A betrifft einen flächigen elektrochemisch arbeitenden Sauerstoffsensor. In einer Ausführungsform umfasst der "Sensor" eine Probenkammer, durch die bspw. Blut fließen kann. Weiterhin weist der Sensor verschiedene Elektroden zur Messung auf, die über eine Membran von einer Probenkammer getrennt sind.

Die Druckschrift US 4 734 184 A bezieht sich auf einen Elektroden- oder Mikroelektrodensensorapparat zur Messung verschiedener Charakteristiken einer Blutprobe mit einer Einweg-Kassette, die einen oder mehrere Sensoren enthält.

Es ist eine Aufgabe der vorliegenden Erfindung, ein Zwischenelement für eine medizintechnische extrakorporale Fluidleitung bereitzustellen, bei welchem eine Fluidgasanalyse, wie z.B. eine Blutgasanalyse (BGA), auf effiziente Weise vorgenommen werden kann. Es ist eine weitere Aufgabe der vorliegenden Erfindung, ein medizintechnisches extrakorporales Fluidsystem, insbesondere einen medizintechnischen extrakorporalen Blutkreislauf, zur Verfügung zu stellen, der eine effiziente Fluidgasanalyse, insbesondere eine effiziente Blutgasanalyse, erlaubt.

Die Erfindung geht aus von einem Zwischenelement für eine medizintechnische extrakorporale Fluidleitung, die dazu eingerichtet ist, Blut zu führen, mit einem Basiskörper, der sich zwischen Verbindungsteilen erstreckt, wobei Basiskörper und Verbindungsteile durchgehend von einem Durchflusskanal zur Führung des Fluid durchsetzt sind und wobei die Verbindungsteile dazu eingerichtet sind, den Basiskörper hydraulisch mit der Fluidleitung zu verbinden und wobei peripher am Basiskörper eine Aufnahme angeordnet ist, die dazu eingerichtet ist, einen Messwertgeber aufzunehmen und wobei an der Aufnahme im Basiskörper ein Durchbruch zum Durchflusskanal angeordnet ist, der über ein elastisches Element zur Aufnahme hin fluiddicht verschlossen ist.

Dabei ist erfindungsgemäß vorgesehen, dass die Aufnahme dazu eingerichtet ist, einen als Gassensor ausgebildeten Messwertgeber einer Sensoreinrichtung zur Messung wenigstens eines in dem Blut enthaltenen Gases aufzunehmen kann und dass das elastische Element ein Diffusionselement ist, welches permeabel für wenigstens ein Gas ist, wobei das Diffusionselement in einem Verbindungsbereich stoffschlüssig mit einem Rand des Durchbruchs verbunden ist und als Diffusionsfolie von kreisrunder Geometrie ausgebildet und zur Aufnahme hin vorgespannt ist. Der Rand kann dabei z.B. als umlaufender Kragen ausgebildet sein, der ringförmig um den Durchbruch herum am Basiskörper angeordnet ist. Das Diffusionselement kann dabei z.B. durch ein Schweiß- oder Klebeverfahren mit einer ringförmigen, radial außenliegenden Zone des Diffusionselementes fluiddicht verbunden sein.

Durch diese Maßnahme kann an dem Zwischenelement ein Gassensor angeordnet werden, durch welchen eine Blutgasanalyse möglich ist, wenn der Durchflusskanal im Zwischenelement von Blut durchflossen ist. Hierdurch wird bei der Verwendung des erfindungsgemäßen Zwischenelementes in medizintechnischen extrakorporalen Fluidsystemen, wie z.B. medizintechnischen extrakorporalen Blutkreisläufen, eine Inline-Blutgasanalyse ermöglicht, die in Echtzeit während der Anwendung des Systems erfolgt. Dieses ist von Vorteil für die Regelung/Steuerung eines medizintechnischen extrakorporalen Fluidsystems, wie z.B. eines medizintechnischen extrakorporalen Blutkreislaufs.

Durch die Anwendung eines gasdurchlässigen Diffusionselements in der Naht zwischen dem Durchflusskanal im Basiskörper des Zwischenelements und der Aufnahme für den Gassensor, wird ein direkter Kontakt des Gassensors mit dem im Durchflusskanal geführten Blut vermieden, so dass der Gassensor ohne aufwendige Reinigungsarbeiten weiterverwendet werden kann. Als Gassensor kann z.B. ein für die transkutane CO₂/O₂-Messung verwendbarer Gassensor verwendet werden.

Erfindungsgemäß ist das Diffusionselement als Diffusionsfolie ausgebildet. Eine derartige Diffusionsfolie kann produktionstechnisch leicht in die gewünschte Form geschnitten werden und in einfacher Weise mit dem Zwischenelement verbunden werden. Die Diffusionsfolie ist von kreisrunder Geometrie, wodurch asymmetrische Spannungen, und Faltenbildungen beim Anbringen der Diffusionsfolie in der Aufnahme des Zwischenelements leicht zu vermeiden sind.

In einer vorteilhaften Weiterbildung des Diffusionselements bzw. der Diffusionsfolie besteht diese aus einem Material der Gruppe der Polymethylpentene, hierbei vorzugsweise aus Poly-4-Methyl-1-Penten. Dieses Material ist sehr gut permeabel für Gase wie CO₂ oder O₂, jedoch absolut flüssigkeitsdicht, was einen Durchtritt von Blut oder anderen Körperfluiden durch das Diffusionselement verhindert. Alternativ kann z.B. auch Polypropylen als Material des Diffusionselements bzw. der Diffusionsfolie Verwendung finden, welches ebenfalls hervorragende Gasdiffusionseigenschaften besitzt und gegenüber Flüssigkeiten absolut dicht ist.

Ferner kann es vorgesehen sein, dass die den Durchflusskanal begrenzende Wandung mit einer Beschichtung zur Verhinderung oder Hemmung der Koagulation und/oder von Immunreaktionen ausgerüstet ist. Die den Durchflusskanal begrenzende Wandung kann z.B. mit Heparin, insbesondere mit hochmolekularem Heparin, beschichtet sein. Hierdurch wird eine Optimierung der Hämokompabilität des Zwischenelements erreicht. Es könnte z.B. auch eine Albumin-Beschichtung vorgesehen sein, zur Verhinderung einer Immunreaktion, oder andere künftig verfügbare Beschichtungen.

In einer weiteren günstigen Fortbildung der Erfindung kann das Diffusionselement wenigstens an seiner, dem Durchflusskanal zugewandten Seite, mit einer Beschichtung zur Verhinderung oder Hemmung der Koagulation und/oder von Immunreaktionen ausgerüstet ist. Das Diffusionselement kann z.B. wenigstens an seiner, dem Durchflusskanal zugewandten Seite, mit Heparin, insbesondere mit hochmolekularem Heparin, beschichtet sein. Auch durch diese Maßnahme wird eine Optimierung der Hämokompabilität des Diffusionselements und damit des Zwischenelements erreicht. Es könnte z.B. auch eine Albumin-Beschichtung vorgesehen sein, zur Verhinderung einer Immunreaktion, oder andere künftig verfügbare Beschichtungen.

Erfindungsgemäß ist das Diffusionselement, insbesondere die Diffusionsfolie, zur Aufnahme hin vorgespannt. Die Vorspannung kann vom Fachmann in Abhängigkeit davon eingestellt werden, ob das Zwischenelement mit dem Sensor vor oder hinter dem Gasaustauschmodul bzw. dem Oxygenator angebracht ist, bzw. ob das Zwischenelement vor oder nach einer Pumpe angeordnet ist, ob es also unter Überdruck oder Unterdruck steht. Durch diese Maßnahme wird erreicht, dass der Gassensor, in, in der Aufnahme angeordnetem Zustand, direkt an dem Diffusionselement ohne Naht anliegt. Das Einbringen einer Flüssigkeit zwischen dem Diffusionselement und dem Gassensor kann damit entfallen.

In einer weiteren Fortbildung der Erfindung, ist der Übergang vom Diffusionselement zur angrenzenden Wandung des Durchflusskanals hin strömungsglatt ausgebildet. D.h. im Übergang sind keine Kanten bzw. Stufen vorhanden, bzw. sind diese minimal. Durch diese Ausbildung können Verwirbelungen, insbesondere bei Durchflusskanaldurchmessern bzw. Fluidleitungsdurchmessern von 3/8 Zoll oder 1/4 Zoll vermieden werden. Derartige Verwirbelungen könnten ansonsten zu Ablagerungen, insbesondere Blutgerinnseln, führen.

Am Basiskörper des Zwischenelements im Bereich der Aufnahme kann eine Halteeinrichtung vorgesehen sein, die dazu eingerichtet ist, den Gassensor reversibel an der Aufnahme festzulegen. Hierdurch kann der Gassensor lösbar am Zwischenelement festgelegt werden und ebenso leicht wieder entfernt werden.

Vorzugsweise weist die Halteeinrichtung am Zwischenelement wenigstens ein Halteelement auf, welches dazu ausgebildet ist, mit einem Gegenhalteelement des Gassensors zu kooperieren. Insbesondere kann das Halteelement mit dem Gegenhaltelement in Eingriff gebracht werden kann, so dass der Gassensor lösbar in der Aufnahme am Zwischenelement festgelegt werden kann.

Das wenigstens eine Haltelement ist dabei vorzugsweise als Rasthaken ausgebildet, der dazu eingerichtet ist, mit einem als Rastvorsprung ausgebildeten Gegenhalteelement des Gassensors zu kooperieren. Hierdurch kann eine Rastverbindung bzw. Schnappverbindung erzeugt werden, über welche der Gassensor in einfacher Weise reversibel in der Aufnahme des Zwischenelements fixierbar ist. Zum sicheren Fixieren des Gassensors in der Aufnahme des Zwischenelements kann die Halteeinrichtung mehrere Haltelemente aufweisen, die umfänglich um die Aufnahme herum angeordnet sind. Alle oder wenigstens eines der Halteelemente können dabei als Rasthaken ausgebildet sein.

In einer vorteilhaften Ausbildung der Erfindung ist das Zwischenelement, mit dem darin angeordneten Diffusionselement, als Einwegelement, bzw. Einwegprodukt, ausgeführt, welches nur zu einer einmaligen Benutzung ausgelegt ist. Durch diese Maßnahme kann eine hygienisch einwandfreie Lösung bereitgestellt werden, die auf eine umweltbelastende Desinfizierung und Reinigung des Zwischenelements nach Gebrauch bewusst verzichtet.

Ein derartiges Einwegprodukt kann insbesondere in sterilen Sicherheitsverpackungen konfektioniert werden.

Die Erfindung geht ferner aus von einem medizintechnischen extrakorporalen Fluidsystem, insbesondere einem medizintechnischen extrakorporalen Blutkreislauf, mit wenigstens einer Fluidleitung, die dazu eingerichtet ist, Blut zu führen, mit wenigstens einem Einleitelement, das dazu eingerichtet ist, Blut von einem Patienten in die Fluidleitung zu führen, und mit einem Gasaustauschmodul, das hydraulisch mit der wenigstens einen Fluidleitung verbunden ist.

Erfindungsgemäß ist die Fluidleitung dabei dazu eingerichtet, mit einem erfindungsgemäßen Zwischenelement verbunden zu werden, so dass das Zwischenelement hydraulisch in die Fluidleitung zwischengeschaltet ist. D.h., dass Blut, welches durch die Fluidleitung geführt wird, auch durch das Zwischenelement hindurchgeführt wird. Dabei wird das Blut an dem Diffusionselement vorbeigeführt und damit an einem in der Aufnahme befindlichem Gassensor, welcher eine Fluidgas-, insbesondere Blutgasanalyse, in Echtzeit durchführen kann. Die gewonnenen Messdaten können dabei als Eingangsparameter zur Einstellung des medizintechnischen extrakorporalen Fluidsystems, insbesondere des medizintechnischen extrakorporalen Blutkreislaufs, genutzt werden. Von Vorteil kann es dabei sein, wenn eine erste Fluidleitung, die einerseits mit dem Einleitelement und andererseits mit dem Gasaustauschmodul verbunden ist, zur hydraulischen Verbindung mit dem Zwischenelement eingerichtet ist. Diese erste Fluidleitung kann insbesondere eine erste blutführende Leitung sein, über die einem Patienten Blut entnommen wird und zu einem Gasaustauschmodul hingeführt wird. Durch die Möglichkeit, das erfindungsgemäße Zwischenelement mit einem Gassensor in dieser ersten Fluidleitung anzubringen, kann eine Gasanalyse des unbehandelten vom Patienten entnommenen Bluts vorgenommen werden.

Idealerweise weist die erste Fluidleitung dabei zwei voneinander getrennte Leitungsabschnitte auf, deren dem Einleitelement und dem Gasaustauschmodul abgewandte Enden jeweils zur Verbindung mit dem Zwischenelement eingerichtet sind. Die beiden freien Enden der beiden Leitungsabschnitte der ersten Fluidleitung können also jeweils mit einem Verbindungsteil des Zwischenelementes verbunden werden, so dass das Zwischenelement hydraulisch in die Fluidleitung zwischen eingeschaltet werden kann.

Im Anwendungszustand des medizintechnischen extrakorporalen Fluidsystems ist das Zwischenelement in die erste Fluidleitung hydraulisch zwischengeschaltet. Ist der Gassensor an diesem Zwischenelement angeordnet, dann kann eine Fluidgasanalyse bzw. eine Blutgasanalyse des durch die erste Fluidleitung strömenden Fluids bzw. Bluts erfolgen.

Die vorliegende Beschreibung offenbart ferner ein Verfahren zum Messen eines in einem medizintechnischen extrakorporalen Fluidsystem, insbesondere einem medizintechnischen extrakorporalen Blutkreislauf, geführten Fluid des menschlichen oder tierischen Körpers enthaltenen Gases. Bei diesem Verfahren wird zunächst in einer Fluidleitung des medizintechnischen extrakorporalen Fluidsystems ein erfindungsgemäßes Zwischenelement hydraulisch zwischengeschaltet. D.h., das erfindungsgemäße Zwischenelement wird mittels seiner beiden Verbindungsteile mit den beiden freien Enden der ersten, zwei Leitungsabschnitte umfassenden, Fluidleitung verbunden. Demnach wird an der Aufnahme des Zwischenelements ein Gassensor einer Sensoreinrichtung angeordnet. Weiterhin wird ein Fluid des Patienten in eine erste Fluidleitung des Fluidsystems geführt und über eine weitere Fluidleitung wieder dem Patienten zugeführt. Dabei strömt das Fluid durch einen Durchflusskanal des Zwischenelements, wobei es an dem Diffusionselement vorbeigeführt wird, das an seiner dem Durchflusskanal abgewandten Seite an dem Gassensor, insbesondere an einem Messfühler des Gassensors, anliegt. Der Gassensor misst dabei den Partialdruck wenigstens eines, in dem Fluid enthaltenen Gases aus der Gruppe O₂, CO₂, Co, N₂. Aus den Partialdruckdaten kann die Konzentration der Gase im Fluid bestimmt werden. Durch das erfindungsgemäße Verfahren können Fluidgasdaten, insbesondere Blutgasanalysedaten, in Echtzeit während der Betriebszeit des medizintechnischen extrakorporalen Fluidsystems gewonnen werden und direkt zur Einstellung des medizintechnischen extrakorporalen Fluidsystems genutzt werden. Die Gasdaten können z.B. zur Überprüfung der Leistungsdaten der Oxygenierung und der CO₂-Eliminierung dienen. Diese Parameter können dann zur Einstellung der Oxygenierung über den Blutfluss und zur Einstellung der CO₂-Eliminierung über die Gasrate.

In einem weiteren Schritt übermittelt der Gassensor die Messdaten über eine Datenleitung an eine Auswerteeinheit der Sensoreinrichtung. Die Messdaten können der Auswerteeinheit entnommen werden und wieder zur Einstellung und Optimierung der Funktion des medizintechnischen extrakorporalen Fluidsystems genutzt werden.

Insbesondere ist das Fluid Blut, welches aus einem Gefäß eines Patienten, die eine Arterie oder Vene sein kann, in die Fluidleitung des Fluidsystems geführt wird, wobei das Blut aus dem Fluidsystem über eine weitere Fluidleitung wieder in eine Ader des Patienten, die eine Arterie sein kann, eingeleitet wird.

Je nach der Art des medizintechnischen extrakorporalen Fluidsystems können z.B. als Kanülen ausgebildete Einleitelemente bzw. Ausleitelemente in die Arterie oder Vene eingeführt werden, um dort Blut aus- oder einzuleiten.

Bei einer Ausgestaltung des medizintechnischen extrakorporalen Fluidsystems als medizintechnischer extrakorporaler Blutkreislauf, insbesondere für eine extrakorporale Lungenunterstützung (ECMO bzw. ECLA), sind die Zugangswege über die das Blut des Patienten entnommen und dem Patienten wiederzugeführt werden, z.B. die *Vena femoralis* und die *Vena jugularis.*

Im Falle einer extrakorporalen Lungenunterstützung durch ein pumpenloses extrakorporales Lungenunterstützungssystem (ILA oder PECLA) kann die Blutentnahme z.B. über die *Arteria femoralis* und die Blutrückführung über die *Vena femoralis* erfolgen.

Als Fluidleitung im Sinne der Erfindung ist dabei bevorzugt jede Art von Leitung zu verstehen, welche in Verbindung mit einer medizinischen Versorgung, Diagnose oder Therapie genutzt werden kann, z.B. auch in Verbindung mit irgendwelchen Kathetern. Die insbesondere blutführende Fluidleitung kann Teil eines sogenannten Schlauchsets sein, oder dieses Schlauchset bilden. Die Fluidleitung kann z.B. als Schlauchleitung aus einem zumindest teilelastischen Material sein. Bevorzugt ist die Fluidleitung flexibel, also elastisch formbar. Insbesondere kann die Fluidleitung gekrümmt oder gebogen werden. Der Durchmesser der Fluidleitung kann weitgehend frei gewählt werden. Zweckdienlich sind insbesondere Außendurchmesser von z.B. 3/8 Zoll oder 1/4 Zoll.

Die Wandung der Fluidleitung oder auch die gesamte Fluidleitung kann aus einem flexiblen Kunststoffmaterial, insbesondere aus Polyvinylchlorid (PVC)-Material, ausgeführt sein. Bevorzugt ist das Kunststoffmaterial ein hochreines, phtalatfreies Weich-PVC. Im einfachsten Fall handelt es sich bei der Schlauchleitung z.B. um einen in der Medizintechnik häufig eingesetzten PVC-Schlauch. Eine Wandstärke der Wandung und/oder Fluidleitung liegt dabei z.B. im Bereich von 1 mm bis 5 mm, bevorzugt im Bereich von 1,2 mm bis 3,5 mm, weiter bevorzugt im Bereich von 1,5 mm bis 3 mm, insbesondere im Bereich von 1,6 mm bis 2,4 mm.

Das Zwischenelement ist bevorzugt aus einem Kunststoffmaterial, insbesondere als Kunststoffspritzgussteil, hergestellt. Als Material für das Zwischenelement kommt z.B. Polycarbonat in Frage.

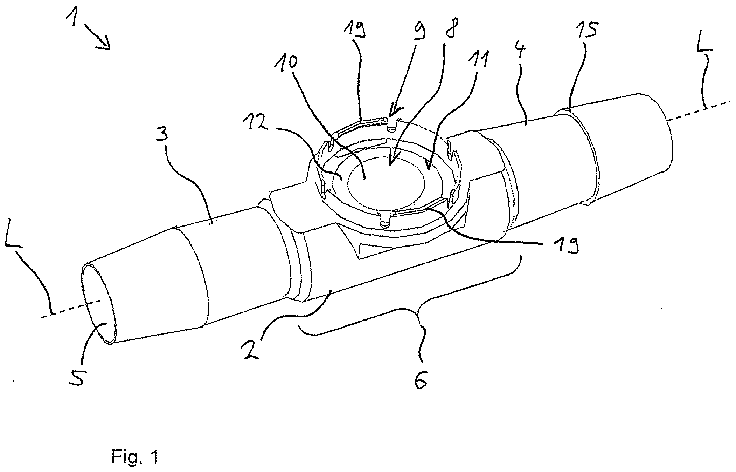

In den nachfolgenden Figuren wird die Erfindung anhand von Ausführungsbeispielen näher erläutert. Im Zusammenhang mit der Beschreibung wird bei einzelnen Bezugszeichen, sofern diese nicht explizit erläutert werden, auf das Ausführungsbeispiel der Figur 1 oder auf die weiteren Figuren verwiesen. Es zeigen:
Figur 1 In schematischer Darstellung in perspektivischer Ansicht ein Zwischenelement gemäß einem Ausführungsbeispiel der Erfindung;
Figur 2 In schematischer Darstellung in perspektivischer Ansicht das Zwischenelement aus Figur 1 mit in einer Aufnahme am Zwischenelement angeordnetem Gassensor;
Figur 3 Eine schematische Schnittansicht des Zwischenelements mit daran angeordnetem Gassensor in einem Schnitt entlang der Linie III-III aus Figur 2;
Figur 4 In schematischer Darstellung ein medizintechnisches extrakorporales Fluidsystem;
Figur 5 In schematischer Darstellung ein weiteres medizintechnisches extrakorporales Fluidsystem.

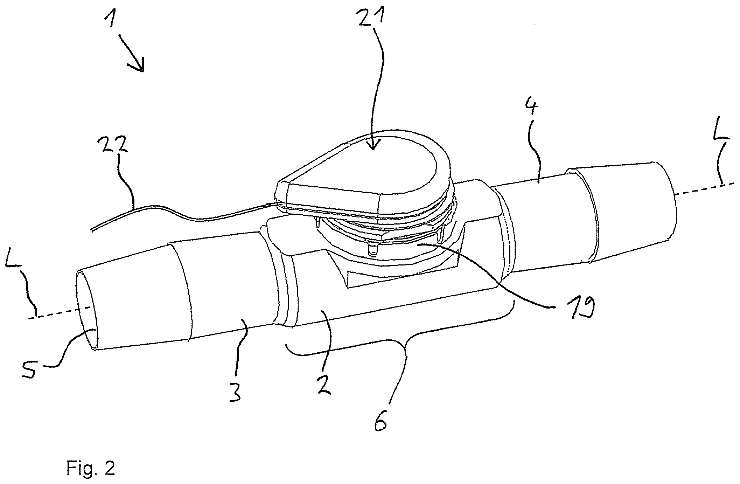

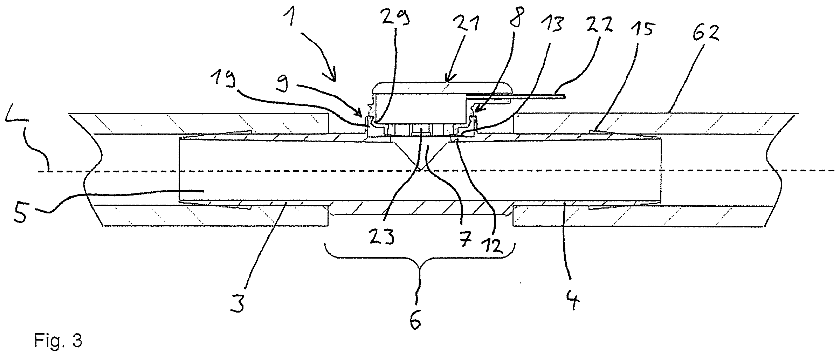

In den Figuren 1 bis 3 ist ein erfindungsgemäßes Zwischenelement 1 für eine medizintechnische extrakorporale Fluidleitung 62 in einem Ausführungsbeispiel wiedergegeben. Das Zwischenelement 1 dient insbesondere der fluiddichten Verbindung von zwei Schlauchabschnitten und weist eine insgesamt längsgestreckte, sich an einer Längsachse L orientierende Form auf und umfasst einen Basiskörper 2, an dem sich in beiden Längsrichtungen der Längserstreckung L Verbindungsteile 3, 4 anschließen. Der Basiskörper 2 bildet dabei einen zentralen Abschnitt 6. Radial außen am Basiskörper 2 ist eine Aufnahme 8 angeordnet, die in dem vorliegenden Ausführungsbeispiel eine kreisrunde Ausgestaltung hat und die radial außen durch Halteelemente 19 einer Halteeinrichtung 9 für einen Gassensor 21, der in den Figuren 2 und 3 dargestellt ist, begrenzt wird. Die Halteelemente 19 der Halteeinrichtung 9 sind dabei ringförmig um die Aufnahme 8 herum angeordnet.

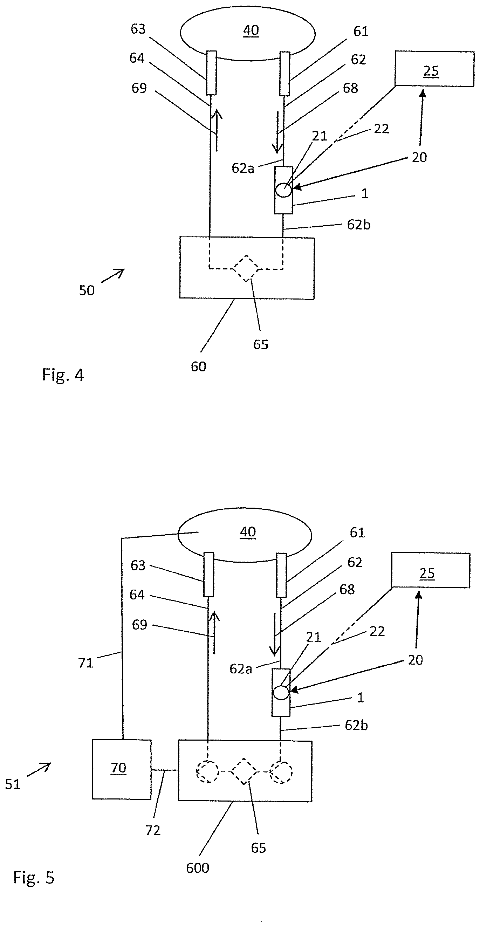

Der Basiskörper 2 und die beiden Verbindungsteile 3 und 4 werden in axialer Richtung entlang der Längserstreckung L von einem durchgehenden Durchflusskanal 5 durchsetzt, der radial außen durch eine Wandung 14 im Basiskörper 2 und den Verbindungsteilen 3, 4 begrenzt wird. Der Basiskörper 2 weist dabei abseits der Aufnahme 8 einen Radius auf, der maximal dem dreifachen des Innenradius des Durchflusskanals 5 entspricht. Das heißt, der Basiskörper 2 ist senkrecht zu seiner Längserstreckung L nur unwesentlich grösser, als die durch ihn miteinander zu verbindenden Leitungsabschnitte bzw. Schlauchleitungsabschnitte der Fluidleitung 62, 64. Im Bereich der Aufnahme 8 ist im Basiskörper 2 bzw. der dortigen Wandung 14 ein Durchbruch 7 angeordnet, der insbesondere in Figur 3 deutlich sichtbar ist. Dieser Durchbruch, welcher in dem vorliegenden Ausführungsbeispiel durch eine kreisrunde Öffnung gebildet wird, ist zur Aufnahme 8 hin über ein Diffusionselement 10 verschlossen. Das Diffusionselement 10 hat vorliegend eine kreisrunde Form. Das Diffusionselement 10 ist dabei in einem radial außenliegenden Verbindungsbereich 11 stoffschlüssig, insbesondere durch Verkleben oder Verschweißen, mit einem ringförmigen Randbereich 13 des Diffusionselements 10 an einem ebenfalls ringförmigen Randbereich 12, der insbesondere als Kragen ausgebildet ist, an dem Basiskörper 2 des Zwischenelements 1 fixiert. Diese stoffschlüssige Verbindung zwischen Diffusionselement 10 und Zwischenelement 1 ist dabei absolut fluiddicht. Wie aus den Figuren 2 und 3 ersichtlich, kann an der Aufnahme 8 ein Gassensor 21 einer Sensoreinrichtung 20 reversibel, d.h. lösbar, festgelegt werden. Diesem reversiblen Festlegen des Gassensors 21 an der Aufnahme 8 dient die bereits vorbezeichnete Halteeinrichtung 9, die die in dem vorliegenden Beispiel ringförmig um die Aufnahme 8 herum angeordneten Halteelemente 19 aufweist. Diese Halteelemente 19 sind vorliegend als Rasthaken ausgebildet, die mit als Gegenrastelementen ausgebildeten Gegenhalteelementen 29 an dem Gassensor 21 in Eingriff bringbar sind. Die Halteeinrichtung 9 fungiert dabei als eine Art Rast- bzw. Schnappverbindung, über die der Gassensor auf einfache Weise schnell an dem Zwischenelement 1 festgelegt werden kann und auch schnell von diesem wieder entfernt werden kann. Wie insbesondere den Figuren 2 und 3 zu entnehmen ist, ist an dem Gassensor 21 eine Messdatenleitung 22 angeordnet, über die der Gassensor 21 Messdaten mit einer Auswerteeinheit 25 (nur in den Figuren 4 und 5 wiedergegeben) verbunden ist und dieser Messdaten im Betrieb der Sensoreinrichtung 20 übermitteln kann. Das Diffusionselement 10 ist vorliegend als Diffusionsfolie ausgebildet und besteht aus Poly-4-Methyl-1-Penten. Es kann aber alternativ z.B. auch aus Polypropylen oder aus einem anderen Material aus der Gruppe der Polymethylpentene hergestellt sein. Das Diffusionselement 10 weist in dem vorliegenden Ausführungsbeispiel eine Materialstärke von 0,1 mm bis 2,0 mm, vorzugsweise von 0,05 ± 0,01 mm auf. Das Zwischenelement 1 selbst ist vorzugsweise aus einem Kunststoff hergestellt, wie z.B. aus einem Kunststoff der Gruppe Polycarbonate.

Der Übergang von dem, den Durchbruch 7 ringförmig umgebenden Randbereich 12 zum Diffusionselement 10, ist in dem vorliegenden Beispiel strömungsglatt ausgebildet, d.h. in diesem Übergangsbereich befinden sich keine bzw. nur unwesentliche Kanten bzw. Stufen, die den Fluss einer Fluidströmung in irgendeiner Weise behindern oder beinträchtigen könnten bzw. zu einer Wirbelbildung führen könnten. Ebenfalls wird hierdurch verhindert, dass es in diesem Übergangsbereich zu Ablagerungen kommen kann.

Sowohl die Wandung 14, im gesamten Bereich des Durchflusskanals 5, als wie auch die dem Durchflusskanal 5 zugewandte Seite des Diffusionselements 10 können mit einer Beschichtung zur Verhinderung oder Hemmung der Koagulation und/oder von Immunreaktionen ausgerüstet sein.

Die dem Durchflusskanal begrenzende Wandung 14 und/oder die dem Durchflusskanal 5 zugewandte Seite des Diffusionselements 10 können mit Heparin, insbesondere mit hochmolekularem Heparin, beschichtet sein. Ferner könnte z.B. alternativ oder auch zusätzlich eine Albuminbeschichtung vorgesehen sein, zur Verhinderung einer Immunreaktion. Wie bereits erwähnt, könnten aber auch andere künftig verfügbare Beschichtungen vorgesehen sein.

Wie insbesondere aus Figur 3 ersichtlich ist, liegt die Unterseite des Gassensors 21, d.h. die Seite, die dem Durchflusskanal 5 bzw. dem Diffusionselement 10 zugewandt ist, spaltfrei an dem Diffusionselement 10 an. In dem vorliegenden Ausführungsbeispiel ist dazu das Diffusionselement 10 zur Aufnahme 8 hin vorgespannt. Durch die Vorspannung entsteht zwischen dem Diffusionselement 10 und dem Gassensor 21 also eine eng anliegende mechanische Verbindung ohne Luftspalt.

Zu bemerken ist, dass der Gassensor 21 die Aufnahme 8 nur in Richtung der Anschlussstelle der Messdatenleitung 22 überragt, jedoch nicht das Zwischenelement 1 in Richtung seiner Längserstreckung L überragt. Die Gefahr eines unbeabsichtigten Lösens des Gassensors 21 von der Aufnahme 8 bzw. dem Zwischenelement 1 ist dadurch vermieden.

In Figur 3 ist beispielhaft dargestellt, wie ein Ende einer ersten Fluidleitung 62 mit einem Verbindungsteil 3 des Zwischenelements 1 kooperiert. Das Ende der Fluidleitung 62 ist dabei außen auf das Verbindungsteil 3 aufgeschoben und wird von einem umlaufenden Vorsprung 15 am Verbindungsteil 3 an diesem gehalten.

In Figur 4 ist ein erstes Ausführungsbeispiel eines medizintechnischen extrakorporalen Fluidsystems 50 dargestellt, welches z.B. als pumpenloses arterio-venöses Lungenunterstützungssystem (PECLA) oder als interventionelle extrakorporale Lungenunterstützung (ILA) ausgebildet ist. Derartige Systeme stellen extrakorporale Blutkreisläufe dar, in denen ein Fluid, nämlich Blut, aus dem Körper eines Patienten 40 herausgeführt, durch ein Gasaustauschmodul 60 hindurchgeführt und dem Patienten 40 wieder zugeführt wird. In dem Gasaustauschmodul 60 wird dabei dem Fluid bzw. Blut CO₂ entzogen und das Blut gleichzeitig mit Sauerstoff bzw. O₂ angereichert. Bei dem in Figur 4 dargestellten medizintechnischen extrakorporalen Fluidsystem 50 wird einem Patienten 40 über ein Einleitelement 61, welches insbesondere als Kanüle ausgebildet ist, ein Fluid, nämlich Blut, entnommen und in eine erste Fluidleitung 62 eingespeist. Diese erste Fluidleitung 62 weist dabei einen ersten Leitungsabschnitt 62a und einen weiteren Leitungsabschnitt 62b auf. Der erste Leitungsabschnitt 62a verläuft dabei von dem Einleitelement 61 bis zu einem erfindungsgemäßen Zwischenelement 1, welches über die in den Figuren 1 bis 3 dargestellten Verbindungsteile 3 und 4 mit den beiden freien Enden der Leitungsabschnitte 62a und 62b mit der ersten Fluidleitung 62 verbunden ist. D.h., ein Blutfluss in Strömungsrichtung 68 verläuft vom ersten Leitungsabschnitt 62a der ersten Fluidleitung 62 durch das Zwischenelement 1 hindurch in den zweiten Leitungsabschnitt 62b der ersten Fluidleitung 62 hin zu einem Gasaustauschelement 65 im Gasaustauschmodul 60. Diese Gasaustauschelement 65 kann z.B. ein Oxygenator sein. Über dem an dem Zwischenelement befindlichen Gassensor, wie er insbesondere in den Figuren 2 und 3 dargestellt ist, wird im Betrieb des medizintechnischen extrakorporalen Fluidsystems 50 der Partialdruck wenigstens eines Gases aus der Gruppe O₂, CO₂, CO, N₂ im Blut gemessen. Aus den Partialdruckdaten kann dann die Konzentration der Gase im Blut bestimmt werden.

Von dem Gasaustauschelement 65 geht eine weitere Fluidleitung 64 ab, die an ihrem, dem Gastauschelement 65 abgewandten, Ende mit einem Ausleitelement 63, wie z.B. einer Kanüle verbunden ist, über das, das in der Fluidleitung 64 in Strömungsrichtung 69 rückgeführte Blut in den Körper des Patienten 40 zurückgeführt wird. Das Ausleitelement 63 wird dabei insbesondere in eine Vene, insbesondere die *Vena femoralis* eingeführt. Das Einleitelement 61 hingegen ist vorzugsweise in einer Arterie, insbesondere die *Arteria femoralis* eingeführt. CO₂ und O₂ können aber generell an Venen und Arterien gemessen werden.

In Figur 5 ist ein weiteres medizintechnisches extrakorporales Fluidsystem 51 dargestellt. Das dargestellte Fluidsystem 51 ist z.B. ein System zur extrakorporalen Kreislaufunterstützung bzw. extrakorporalem Lifesupport (ECLS). Auch bei diesem medizintechnischen extrakorporalen Fluidsystem 51 wird einem Patienten 40 über ein Einleitelement 61, welches insbesondere als Kanüle ausgebildet ist, ein Fluid, nämlich Blut, entnommen und in eine erste Fluidleitung 62 eingespeist. Diese erste Fluidleitung 62 weist dabei wiederum einen ersten Leitungsabschnitt 62a und einen weiteren Leitungsabschnitt 62b auf. Der erste Leitungsabschnitt 62a verläuft dabei von dem einen Leitelement 61 bis zu einem erfindungsgemäßen Zwischenelement 1, welches über die in den Figuren 1 bis 3 dargestellten Verbindungsteile 3 und 4 mit den beiden freien Enden der Leitungsabschnitte 62a und 62b mit der ersten Fluidleitung 62 verbunden ist. Wie bei dem Ausführungsbeispiel aus Figur 4 erfolgt auch hier ein Blutfluss in Strömungsrichtung 68 vom ersten Leitungsabschnitt 62a der ersten Fluidleitung 62 durch das Zwischenelement 1 hindurch in den zweiten Leitungsabschnitt 62b der ersten Fluidleitung hin. Das Gasaustauschmodul 600 weist in dem vorliegenden Ausführungsbeispiel neben dem Gasaustauschelement 65 noch eine Pumpe 66 auf, zu der der zweite Leitungsabschnitt 62b der ersten Fluidleitung 62 hinführt. Diese Pumpe 66 ist dann über eine weitere Leitung mit dem Gasaustauschelement 65, wie z.B. einem Oxygenator, hydraulisch verbunden. Dem Gasaustauschelement 65 ist im Fluidkreislauf dann noch eine weitere Pumpe 67 nachgeschaltet, die pumpenausgangsseitig mit der weiteren Fluidleitung 64 verbunden ist, um das Fluid in Strömungsrichtung 69 zum Ausleitelement 63, wie z.B. einer Kanüle, zu fördern und zu dem Patienten 40 zurückzuführen. Bei diesem medizintechnischen extrakorporalen Fluidsystem 51 ist ferner noch eine Steuereinheit 70 vorgesehen, die über eine der Steuerung dienende Leitung 72 mit dem Gasaustauschmodul 600 verbunden ist und über die die Pumpenleistung der Pumpen 66 und 67 gesteuert wird. Über eine weitere Leitung 71 ist die Steuereinheit mit einem EKG (Elektrokardiogramm, Elektrokardiographie) verbunden, welches ein Steuersignal für die Steuereinheit 70 bereitstellt.

Bei Pumpsystemen ist aber auch eine veno-venöse Variante möglich, z.B. bei einer venovenösen extrakorporalen Membranoxygenierung (VV-ECMO). Dies wird z.B. durch eine Mehrfach- bzw. Doppellumenkanüle z.B. in der *Vena jugularis* realisiert. Dabei wäre eine Messung vor und nach dem Gasaustauschelement trotzdem möglich.

Das Zwischenelement könnte z.B. auch als Oxygenator ausgebildet sein und den Sensor enthalten.

### Bezugszeichenliste

- 1: Zwischenelement
- 2: Basiskörper
- 3: Verbindungsteil
- 4: weiteres Verbindungsteil
- 5: Durchflusskanal
- 6: zentraler Abschnitt
- 7: Durchbruch
- 8: Aufnahme (für Gassensor)
- 9: Halteeinrichtung (für Gassensor)
- 10: Diffusionselement
- 11: Verbindungsbereich von 10 an 1
- 12: Randbereich (Kragen)
- 13: Randbereich von 10
- 14: Wandung
- 15: Vorsprung

- 19: Haltelemente

- 20: Sensoreinrichtung
- 21: Gassensor
- 22: Messdatenleitung
- 23: Einlassöffnung von 20
- 24: Messfühler
- 25: Auswerteeinheit

- 29: Gegenhaltelement

- 40: Patient

- 50: Fluidsystem
- 51: weiteres Fluidsystem
- 60: Gasaustauschmodul
- 61: Entnahmeelement (Kanüle)
- 62: erste Fluidleitung
- 62a: erster Leitungsabschnitt
- 62b: weiterer Leitungsabschnitt
- 63: Ausleitelement (Kanüle)
- 64: weitere Fluidleitung
- 65: Gasaustauschelement
- 66: Pumpe
- 67: weitere Pumpe
- 68: Strömungsrichtung
- 69: Strömungsrichtung
- 70: Steuereinheit
- 71: Leitung (für EKG)
- 72: Leitung (70 zu 60)

- 600: weiteres Gasaustauschmodul

- L: Längsachse

## Patentansprüche

1. Zwischenelement (1) für eine medizintechnische extrakorporale Fluidleitung, die dazu eingerichtet ist, Blut zu führen,
mit einem Basiskörper (2), der sich zwischen Verbindungsteilen (3, 4) erstreckt,
wobei Basiskörper (2) und Verbindungsteile (3, 4) durchgehend von einem Durchflusskanal (5) zur Führung des Blutes durchsetzt sind,
und wobei die Verbindungsteile (3, 4) dazu eingerichtet sind, den Basiskörper (2) zum Leiten des Blutes mit der Fluidleitung zu verbinden,
und wobei peripher am Basiskörper (2) eine Aufnahme (8) angeordnet ist, die dazu eingerichtet ist, einen Messwertgeber aufzunehmen,
und wobei an der Aufnahme (8) im Basiskörper (2) ein Durchbruch (7) zum Durchflusskanal (5) angeordnet ist, der über ein elastisches Element zur Aufnahme (8) hin fluiddicht verschlossen ist,
wobei die Aufnahme (8) dazu eingerichtet ist, einen als Gassensor (21) ausgebildeten Messwertgeber einer Sensoreinrichtung (25) zur Messung wenigstens eines in dem Blut enthaltenen Gases aufzunehmen,
und wobei das elastische Element ein Diffusionselement (10) ist, welches permeabel für wenigstens ein Gas ist,
wobei das Diffusionselement (10) in einem Verbindungsbereich (11) stoffschlüssig mit einem Rand (12) des Durchbruchs (7) verbunden ist,
**dadurch gekennzeichnet, dass**
das Diffusionselement (10) als Diffusionsfolie von kreisrunder Geometrie ausgebildet und zur Aufnahme (8) hin vorgespannt ist.

2. Zwischenelement (1) nach Anspruch 1, **dadurch gekennzeichnet, dass** das Diffusionselement (10) aus einem Material der Gruppe Polymethylpentene und Polypropylene gebildet ist.

3. Zwischenelement (1) nach Anspruch 2, **dadurch gekennzeichnet, dass** das Diffusionselement (10) aus Poly-4-methyl-1-pentene gebildet ist.

4. Zwischenelement (1) nach einem der Ansprüche 1 bis 3, **dadurch gekennzeichnet, dass** wenigstens die Wandung (14) des Durchflusskanals (5) mit einer Beschichtung zur Verhinderung oder Hemmung der Koagulation oder von Immunreaktionen ausgerüstet ist.

5. Zwischenelement (1) nach einem der Ansprüche 1 bis 4, **dadurch gekennzeichnet, dass** das Diffusionselement (10) wenigstens an seiner, dem Durchflusskanal (5) zugewandten Seite mit einer Beschichtung zur Verhinderung oder Hemmung der Koagulation oder von Immunreaktionen ausgerüstet ist.

6. Zwischenelement (1) nach einem der Ansprüche 1 bis 5, **dadurch gekennzeichnet, dass** der Übergang (15) vom Diffusionselement (10) zur angrenzenden Wandung (14) des Durchflusskanals (5) hin strömungsglatt ausgebildet ist.

7. Zwischenelement (1) nach einem der Ansprüche 1 bis 6, **dadurch gekennzeichnet, dass** an der Aufnahme (8) eine Halteeinrichtung (9) vorgesehen ist, die dazu eingerichtet ist, den Gassensor reversibel an der Aufnahme (8) festzulegen.

8. Zwischenelement (1) nach Anspruch 7, **dadurch gekennzeichnet, dass** die Halteeinrichtung (9) wenigstens ein Halteelement (19) aufweist, das dazu ausgebildet ist, mit einem Gegenhalteelement (29) des Gassensors (21) zu kooperieren.

9. Zwischenelement (1) nach Anspruch 7 oder 8, **dadurch gekennzeichnet, dass** das wenigstens eine Halteelement (19) als Rasthaken ausgebildet ist, der dazu ausgebildet ist, mit einem als Rastvorsprung ausgebildeten Gegenhalteelement zu kooperieren.

10. Zwischenelement (1) nach einem der Ansprüche 7 bis 9, **dadurch gekennzeichnet, dass** die Halteeinrichtung (9) mehrere Halteelemente (19) aufweist, die umfänglich um die Aufnahme (8) herum angeordnet sind.

11. Zwischenelement (1) nach einem der Ansprüche 1 bis 10, **dadurch gekennzeichnet, dass** es als Einwegelement ausgeführt ist, das nur zur einmaligen Benutzung ausgelegt ist.

12. Medizintechnisches extrakorporales Fluidsystem
mit wenigstens einer Fluidleitung (62, 64), die dazu eingerichtet ist, Blut zu führen,
mit wenigstens einem Einleitelement (61), das dazu eingerichtet ist, Blut von einem Patienten (40) in die Fluidleitung (62) zu führen,
und mit einem Gasaustauschmodul (60, 600), das hydraulisch mit der wenigstens einen Fluidleitung (62, 64) verbunden ist,
**dadurch gekennzeichnet,**
**dass** in die Fluidleitung (62) ein Zwischenelement (1) nach einem der Ansprüche 1 bis 11 hydraulisch zwischengeschaltet ist.

13. Medizintechnisches extrakorporales Fluidsystem nach Anspruch 12, **dadurch gekennzeichnet, dass** das Fluidsystem eine erste Fluidleitung (62) umfasst, die einerseits mit dem Einleitelement (61) und andererseits mit Gasaustauchmodul (60, 600) verbunden ist und zur hydraulischen Verbindung mit dem Zwischenelement (1) eingerichtet ist.

14. Medizintechnisches extrakorporales Fluidsystem nach Anspruch 13, **dadurch gekennzeichnet, dass** die erste Fluidleitung (62) zwei voneinander getrennte Leitungsabschnitte (62a, 62b) aufweist, deren dem Einleitelement (61) und dem Gasaustauschmodul (60, 600) abgewandte Enden jeweils zur Verbindung mit dem Zwischenelement (1) eingerichtet sind.

## Claims

1. Intermediate element (1) for a medical extracorporeal fluid conduit which is adapted to guide blood, the intermediate element (1) comprising:
a base body (2) extending between connecting parts (3, 4),
wherein the base body (2) and the connecting parts (3, 4) are continuously traversed by a flow channel (5) for guiding the blood,
and wherein the connecting parts (3, 4) are adapted to connect the base body (2) to the fluid conduit for guiding the blood,
and wherein a receptacle (8) is peripherally arranged on the base body (2), which is adapted to receive a measuring transducer,
and wherein an aperture (7) to the flow channel (5) is arranged on the receptacle (8) in the base body (2), which aperture (7) is closed fluid-tightly towards the receptacle (8) by means of an elastic member,
wherein the receptacle (8) is adapted to receive a measuring transducer, configured as a gas sensor (21), of a sensor device (25) for measuring at least one gas contained in the blood,
and wherein the elastic member is a diffusion element (10) permeable to at least one gas,
wherein the diffusion element (10) is materially connected in a connecting region (11) to an edge (12) of the aperture (7),
**characterised in that**
the diffusion element (10) is formed as a diffusion foil of circular geometry and is biased towards the receptacle (8).

2. Intermediate element (1) according to claim 1, **characterised in that** the diffusion element (10) is formed from a material selected from the group consisting of polymethylpentenes and polypropylenes.

3. Intermediate element (1) according to claim 2, **characterised in that** the diffusion element (10) is formed from poly-4-methyl-1-pentene.

4. Intermediate element (1) according to any one of claims 1 to 3, **characterized in that** at least the wall (14) of the flow channel (5) is provided with a coating for preventing or inhibiting coagulation or immune reactions.

5. Intermediate element (1) according to any one of claims 1 to 4, **characterized in that** the diffusion element (10) is provided, at least on its side facing the flow channel (5), with a coating for preventing or inhibiting coagulation or immune reactions.

6. Intermediate element (1) according to any one of claims 1 to 5, **characterised in that** the transition (15) from the diffusion element (10) to the adjacent wall (14) of the flow channel (5) is configured to be smooth in terms of flow.

7. Intermediate element (1) according to any one of claims 1 to 6, **characterized in that** a holding device (9) is provided on the receptacle (8), which is configured to reversibly fix the gas sensor on the receptacle (8).

8. Intermediate element (1) according to claim 7, **characterized in that** the holding device (9) has at least one holding element (19) which is configured to cooperate with a counter-holding element (29) of the gas sensor (21).

9. Intermediate element (1) according to claim 7 or 8, **characterized in that** the at least one holding element (19) is configured as a latching hook which is configured to cooperate with a counter-holding element configured as a latching projection.

10. Intermediate element (1) according to any one of claims 7 to 9, **characterised in that** the holding device (9) comprises a plurality of holding elements (19) which are circumferentially arranged around the receptacle (8).

11. Intermediate element (1) according to any one of the claims 1 to 10, **characterized in that** it is configured as a disposable element which is designed for single use only.

12. Medical extracorporeal fluid system, comprising:
at least one fluid conduit (62, 64) adapted to guide blood,
at least one infusion element (61) which is adapted to direct blood from a patient (40) into the fluid conduit (62), and
and a gas exchange module (60, 600) which is hydraulically connected to the at least one fluid conduit (62, 64),
**characterized in that**
an intermediate element (1) according to any one of claims 1 to 11 is hydraulically interposed in the fluid conduit (62).

13. Medical extracorporeal fluid system according to claim 12, **characterised in that** the fluid system comprises a first fluid conduit (62) which is connected on the one hand to the infusion element (61) and on the other hand to a gas exchange module (60, 600) and which is adapted for hydraulic connection to the intermediate element (1).

14. Medical extracorporeal fluid system according to claim 13, **characterised in that** the first fluid conduit (62) comprises two line sections (62a, 62b) separated from one another, the ends of which facing away from the infusion element (61) and the gas exchange module (60, 600) are each adapted for connection to the intermediate element (1).

## Revendications

1. Élément intermédiaire (1) pour une conduite de fluide extracorporel de technique médicale, qui est conçue pour guider le sang,
présentant un corps de base (2), qui s'étend entre des parties de liaison (3, 4), le corps de base (2) et les parties de liaison (3, 4) étant traversés en continu par un canal d'écoulement (5) pour le guidage du sang
et les parties de liaison (3, 4) étant conçues pour relier le corps de base (2) à la conduite de fluide pour guider le sang,
et un logement (8) étant agencé en périphérie du corps de base (2), lequel logement est conçu pour recevoir un transmetteur,
et un passage (7) vers le canal d'écoulement (5) étant disposé au niveau du logement (8) dans le corps de base (2), lequel passage est fermé de manière étanche aux fluides par rapport au logement (8) via un élément élastique,
le logement (8) étant conçu pour recevoir un transmetteur d'un dispositif capteur (25) conçu comme capteur de gaz (21) pour mesurer au moins un gaz contenu dans le sang
et l'élément élastique étant un élément de diffusion (10) qui est perméable à au moins un gaz,
l'élément de diffusion (10) étant relié dans une zone de liaison (11) par liaison de matière à un bord (12) du passage (7), **caractérisé en ce que** l'élément de diffusion (10) est conçu comme une feuille de diffusion de géométrie circulaire et prétendu vers le logement (8).

2. Élément intermédiaire (1) selon la revendication 1, **caractérisé en ce que** l'élément de diffusion (10) est formé d'un matériau du groupe constitué par le polyméthylpentène et le polypropylène.

3. Élément intermédiaire (1) selon la revendication 2, **caractérisé en ce que** l'élément de diffusion (10) est formé de poly-4-méthyl-1-pentène.

4. Élément intermédiaire (1) selon l'une quelconque des revendications 1 à 3, **caractérisé en ce qu'**au moins la paroi (14) du canal d'écoulement (5) est pourvue d'un revêtement pour empêcher ou inhiber la coagulation ou des réactions immunes.

5. Élément intermédiaire (1) selon l'une quelconque des revendications 1 à 4, **caractérisé en ce que** l'élément de diffusion (10) est pourvu, au moins sur son côté orienté vers le canal d'écoulement (5), d'un revêtement pour empêcher ou inhiber la coagulation ou des réactions immunes.

6. Élément intermédiaire (1) selon l'une quelconque des revendications 1 à 5, **caractérisé en ce que** la transition (15) de l'élément de diffusion (10) vers la paroi adjacente (14) du canal d'écoulement (5) est conçue de manière lisse pour l'écoulement.

7. Élément intermédiaire (1) selon l'une quelconque des revendications 1 à 6, **caractérisé en ce qu'**un dispositif de fixation (9) est prévu au niveau du logement (8), lequel dispositif est conçu pour fixer le capteur de gaz de manière réversible au logement (8).

8. Élément intermédiaire (1) selon la revendication 7, **caractérisé en ce que** le dispositif de fixation (9) présente au moins un élément de fixation (19) conçu pour coopérer avec un contre-élément de fixation (29) du capteur de gaz (21).

9. Élément intermédiaire (1) selon la revendication 7 ou 8, **caractérisé en ce que** ledit au moins un élément de fixation (19) est conçu comme un crochet d'encliquetage, qui est conçu pour coopérer avec un contre-élément de fixation conçu comme une saillie d'encliquetage.

10. Élément intermédiaire (1) selon l'une quelconque des revendications 7 à 9, **caractérisé en ce que** le dispositif de fixation (9) présente plusieurs éléments de fixation (19) qui sont disposés de manière périphérique autour du logement (8).

11. Élément intermédiaire (1) selon l'une quelconque des revendications 1 à 10, **caractérisé en ce qu'**il est conçu comme élément à usage unique, qui n'est conçu que pour une seule utilisation.

12. Système de fluide extracorporel de technique médicale présentant au moins une conduite de fluide (62, 64) qui est conçue pour guider le sang,
présentant au moins un élément d'introduction (61) conçu pour guider le sang d'un patient (40) dans la conduite de fluide (62)
et présentant un module d'échange de gaz (60, 600), qui est relié hydrauliquement à ladite au moins une conduite de fluide (62, 64), **caractérisé en ce qu'**un élément intermédiaire (1) selon l'une quelconque des revendications 1 à 11 est inséré hydrauliquement dans la conduite de fluide (62).

13. Système de fluide extracorporel de technique médicale selon la revendication 12, **caractérisé en ce que** le système de fluide comprend une première conduite de fluide (62) qui est reliée d'une part à l'élément d'introduction (61) et d'autre part au module d'échange de gaz (60, 600) et qui est conçue pour la liaison hydraulique à l'élément intermédiaire (1).

14. Système de fluide extracorporel de technique médicale selon la revendication 13, **caractérisé en ce que** la première conduite de fluide (62) présente deux sections de conduite (62a, 62b) séparées l'une de l'autre, dont les extrémités opposées à l'élément d'introduction (61) et au module d'échange de gaz (60, 600) sont à chaque fois conçues pour la liaison à l'élément intermédiaire (1).
